Europäisches Patentamt

European Patent Office

Office européen des brevets

(11) Publication number: **0 261 362**
**A2**

(12) **EUROPEAN PATENT APPLICATION**

(21) Application number: **87111329.6**

(51) Int. Cl.⁴: **A61K 31/12**

(22) Date of filing: **05.08.87**

The title of the invention has been amended (Guidelines for Examination in the EPO, A-III, 7.3).

(30) Priority: **05.08.86 JP 182757/86**

(43) Date of publication of application:
**30.03.88 Bulletin 88/13**

(84) Designated Contracting States:
**AT BE CH DE ES FR GB GR IT LI LU NL SE**

(71) Applicant: **Eisai Co., Ltd.**
**6-10, Koishikawa 4-chome Bunkyo-ku**
**Tokyo 112(JP)**

(72) Inventor: **Okuyama, Shinichi**
**15-38, Nakayama 3-chome**
**Sendai-shi Miyagi(JP)**
Inventor: **Furuse, Kazumaro**
**3-5, Inokashira 2-chome**
**Mitaka-shi Tokyo(JP)**
Inventor: **Ohsawa, Shigemitsu**
**2286-12, Suehiroco**
**Honjou-shi Saitama(JP)**

(74) Representative: **Hansen, Bernd, Dr.rer.nat. et al**
**Hoffmann, Eitle & Partner Patentanwälte**
**Arabellastrasse 4**
**D-8000 München 81(DE)**

(54) **Ubidecarenone as a wound remedy.**

(57) Ubidecarenone is effective to treat a wound when it is applied to the wound.

EP 0 261 362 A2

## WOUND REMEDY

The present invention relates to a wound remedy comprising ubidecarenone as the effective ingredient. Specifically, the present invention relates to the use of ubidecarenone as the medicine which is utilized in the therapy of wounds in the medical field.

The invention provides the use of ubidecarenone for preparation of a wound remedy and then a method of treating a wound which comprises applying to the wound of a patient ubidecarenone.

Wounds are broadly classified into an incised wound, a contused wound, a punctured wound and the like. It is vital to the therapy of a wound to repair a deficit or separation of the skin and the epithelium of a mucous membrane in an affected region by rapid epithelialization. Accordingly, development of a remedy which can promote quick formation, of good granulation tissue under the skin and rapid epithelialization will be of a great clinical significance.

The inventors of the present invention have made various attempts to remedy a wound by administration of a drug. As a result, they have found that a remarkable therapeutic effect can be obtained by directly applying ubidecarenone to the affected region to effect administration. Based on this finding, the present invention has been completed. The inventors of the present invention previously made an attempt to use ubidecarenone for the therapy of radiodermatitis, ulceration from radiation, decubitus, and burn, and observed a remarkable effect as described in references I) and 2). However, they were not able to anticipate as a matter of course that ubidecarenone might exhibit the same remarkable effect on wounds since they differ in cause and condition from radiodermatitis, ulceration from radiation, decubitus, and burn. However, an unexpectedly remarkable wound healing performance of ubidecarenone has been confirmed as described above. Thus, the present invention has been completed. Accordingly, an object of the present invention is to remedy a wound and the present invention provides a remedy characterized by administering ubidecarenone to an affected region for attaining the above-mentioned object.

I) Okuyama, S and Mishina H.: Principia of cancer therapy. I. Rescue of radiation damage. Sci. Rep. Res. Inst. Tohoku Uni. -C 29: I, I982.

2) Okuyama, S and Mishina H.: Principia of cancer therapy. VI. Application of ubiquinone ointment for intractable radiation ulcers: An expander cytochrome c effect? Sci. Rep. Res. Inst. Tohoku Univ. -C 30: 36, I983.

The present invention will now be described in detail.

The term "wounds" as used in the present specification is interpreted in a broad sense and comprehends the whole kinds of skin injuries caused by trauma. Specifically, the term "wounds" comprehends not only an incised wound and a punctured wound caused by an acute angle external force of a knife, a kitchen knife, or a gimlet, but also a cut wound and a contused wound involving detrition or rupture of the tissue and caused by a dull angle external force, an avulsed wound involving avulsion of subcutaneous tissue or skin and caused by an external force in the tangential direction, and a firearms wound caused by a gunshot.

Ubidecarenone, which is also called ubiquinone or coenzyme $Q_{10}$, and which has been used as a remedy for congestive heart failure, is used in the present invention. Ubidecarenone is extracted from the mitochondrion of a bovine cardiac muscle and is known to be involved in the electron transport system. Therefore, it improves the oxygen utilization coefficient in the cardiac muscle and enables a high ATP production function to be maintained even when the cardiac muscle is in an ischemic state. As a result, it is known that the injury of the cardiac muscle in an ischemic state is mitigated by ubidecarenone to ameliorate the reduced heart contraction function.

However, it has not been known that ubidecarenone administered to the skin heals a wound with a remarkable effect. This has been clarified for the first time by the inventors of the present invention.

Ubidecarenone is a yellow to orange crystalline powder having a melting point of 48 to 52°C and fat-soluble. It is hardly soluble in water and methanol. As described above, it has been perorally administered for amelioration of various symptoms of congestive heart failure.

The characteristic feature of the present invention consists in percutaneous administration of ubidecarenone to a region of a wound as defined hereinbefore.

Ubidecarenone may be directly administered to the skin but is desired to be administered in the form of a preparation adapted to application thereof to the skin. Ubidecarenone may be administered in combination thereof with other drugs such as cytochrome c or urokinase, but the present invention is not limited to such a combined administration.

The amount of ubidecarenone to be used in the remedy of the present invention is preferably 0.05 to 5.0%, more preferably 0.I to 2.0%. An adequate amount of the remedy may be applied in accordance with the size and severity of a wound.

The safety of percutaneous administration of ubidecarenone is high, and skin irritation due to ubidecarenone is minor. For example, the results of primary skin irritation, accumulative irritation, eyelid irritation, phototoxis, sensitization, photosensitization, and patch tests are as shown in Table I.

## Table 1

| Item | Concn. | Solvent | Results & conclusion | |
|---|---|---|---|---|
| Primary skin irritation | 1% | Squalane | 0.1 | lowly irritative |
| | − | " | 0.1 | |
| Accumulative skin irritation | 1% | " | 0.3 | ditto |
| | − | " | 0.3 | |
| Eyelid irritation | 1% | " | | lowly irritative |
| | − | " | | |
| Phototoxis | 1% | " | (−) | lowly phototoxic |
| | 10% | " | (−) | |
| Sensitization | Induction: 5% acetone | | | |
| Challenge (Adjuvante & Patch method) | 5% | acetone | 0/10 | lowly sensitized |
| | 1% | " | 0/10 | |
| | Induction: 10% acetone | | | |
| Photosensitivization | 10% | acetone | 0/5 | |
| Challenge (Adjuvante & Strip method) | 5 | " | 0/5 | |
| | 2 | " | 0/5 | lowly photosensitized |
| | 1 | " | 0/5 | |
| | 0.5 | " | 0/5 | |
| Patch test | 1% | squalane | 0/54 | lowly irritative |
| | − | " | 0/54 | |

In order to provide a preparation adapted to percutaneous administration, an adequate lowly irritant raw material for a preparation may be chosen and blended as the ingredient other than ubidecarenone. For example, glycerin, squalane, cetyl alcohol, yolk phospholipid, glycerin fatty acid ester, etc. may be chosen, and a preparation for percutaneous administration may be produced according to the customary method.

The following Examples will more specifically illustrate the present invention.

Example I (hydrophilic ointment)

```
stearly alcohol      I2.0 wt.%
squalane      6.0
isopropyl myristate      4.0
polyoxyethylene cetyl alcohol ether (20 mol)      3.0
stearic acid      2.0
ubidecarenone      0.5
propylene glycol      6.0
ethylparaben      0.I
butylparaben      0.I
purified water      q. s. ad I00
```

The above ingredients were mixed by the customary method to prepare a homegeneous cream as a remedy according to the present invention.

Example 2

```
squalane      I5.0 wt.%
octyldodecyl myristate      5.0
hardened soybean oil      5.0
propylene glycol monostearate      4.0
glycerol monostearate      I.5
stearic acid      2.0
ubidecarenone      I.0
polyoxyethylene-hardened caster oil (50 mol)      I.0
partially hydrogenated yolk phospholipid      I.0
glycerol      5.0
ethylparaben      0.3
antioxidizing agent      a suitable amount
purified water      g. s. ad I00
```

The above ingredients were mixed by the customary method to prepare a homogeneous cream as a remedy according to the present invention.

Example 3

```
squalane      3.0 wt.%
octyldodecyl myristate      2.0
stearic acid      I.2
glycerol monostearate      I.0
sorbitan monopalmitate      0.5
cetyl alcohol      0.5
cetyl monopalmitate      0.5
polyoxyethylene glycerol monostearate (20 mol)      I.5
methylparaben      0.2
propylene glycol      5.0
xanthan gum      0.05
ubidecarenone      0.3
perfume      a suitable amount
purified water      q. s. ad I00
```

The above ingredients were mixed by the customary method to prepare a homogeneous emulsion as a remedy according to the present invention.

4

Example 4

      partially hydrogenated yolk phospholipid      0.l wt.%
      ubidecarenone      0.l
      Macrogol 400      4.0
      ethyl alcohol      8.0 vol.%
      polyoxyethylene-hardened caster oil (50 mol)      0.8 wt.%
      propylene glycol      2.0
      ethylparaben      0.l
      perfume      a suitable amount
      antioxidizing agent      a suitable amount
      purified water      q. s. ad l00.0 vol.%

The above ingredients except for purified water were homogeneously dissolved by heating and kept at about 60°C. The resulting mixture was added to purified water preliminarily heated to about 60°C while stirring to effect homogeneous mixing. The resulting mixture was cooled to room temperature to prepare a lotion containing ubidecarenone as a remedy according to the present invention.

Example 5

      solid paraffin      l.0 wt.%
      microcrystalline paraffin      7.0
      cetyl alcohol      2.0
      aluminum stearate      l.0
      ubidecarenone      l.0
      liquid paraffin      25.0
      white vaseline      q. s. ad l00.0

The above ingredients were mixed by the customary method to prepare a homogeneous ointment as a remedy according to the present invention.

The effect of the present invention will now be illustrated by the following Experimental Example and Cases. A hydrophilic ointment containing 0.5% of ubidecarenone and obtained in Example I or a lotion obtained in Example 4 was used as the remedy of the present invention.

Experimental Example I

Six male white rabbits having a weight of 2 to 3 kg were shaved in their back regions, where they were well depilated using a depilatory cream. After one day, the animals were anesthetized. A circle of l0 mm in diameter was drawn on the depilated skin with a marking ink. The skin was cut off along the circle with a surgical knife and sterilized with Hibitane solution. Aseptic bandages were put on the cut regions of two rabbits as the control group. The base not containing ubidecarenone was applied to two rabbits as the base control group. A 0.5% ubidecarenone ointment was applied to two rabbits as the experimental therapeutic group. The ointment was applied twice a day in the morning and evening. Observation was made for 8 days.

(Experimental results)

Healing of a wound was promoted in the experimental group treated with the ubidecarenone ointment and the base control group. Particularly, the promotion of healing was remarkable in the experimental group. A clear difference in the promotion of healing was already observed on the 8th day.

Case I: A man of 48 years old was shaving with a safety razor and cut the skin to form a skin deficit of 2×2 mm. After disinfection, he applied the ubidecarenone lotion to the affected region three times a day. The wound was healed with incrustation next morning. This effect was repeatedly confirmed thereafter. In general, healing of the incised wound like this requires 2 to 4 days. Innumerable incised wounds are formed by shaving every time. This is particularly so when shaving is done with a dull razor with a strong force.

Thus, it is apparent that it is very beneficial to use ubidecarenone for this kind of incised wounds.

Case 2: A woman of 78 years old has a fugue due to senile dementia. During loitering about, she fell down violently and hit her left knee to have contused wound. Not only abrasion of cuticle but also deficit of corium was observed. The therapy with a ubidecarenone ointment was started. An antibiotic was administered for 3 days for the purpose of preventing infection. On the fourth day, a good granulation tissue began to be formed and reduction of the wound was apparently observed. On the sixth day, she fell down violently again, and a new abrasion was formed. On the ninth day, the sublimis contused wound was almost healed and the deep wound incrusted, however, she peeled the scab every day. Thus, healing of the wound was obstructed. The new abrasion was obstructive, too. In general, healing of wounds of old people is retarded. Besides this, senile dementia makes the healing more difficult due to unexpected obstruction in the healing step. However, the usefulness of the therapy with a ubidecarenone ointment was confirmed.

Case 3: A female baby of one year old put her finger in her mouth and strongly sucked the finger to form a rhagade having a length of about 5 mm in the middle finger on the side of the palm. Neither rubefaction nor hemorrhage was observed and the rhagade was believed to be ischemic wound. Therefore, a ubidecarenone ointment was applied to the wound in the morning and evening. It was healed in two days.

Case 4: A woman of 43 years old was subjected to ablation of her left breast and clearing of her left axillary lymph node because of cancer of her left breast. About one month after the operation, she was put in our charge for the purpose of radiotherapy. As for the postoperative state of the left breast, a number of skin deficits were observed along the suture line. The therapy with a ubidecarenone ointment was started for fear of incomplete suture by radiotherapy. For the purpose of preventing the progression of the cancerous focus, carmofur was simultaneously used as the carcinostatic. The skin deficit was healed after one week. Thus, radiotherapy was started on the 8th day after she was put in our charge. While tegafur and cyclophosphamide were simultaneously used as the carcinostatics, irradiation with an X-ray of 3,000 R was conducted for 3 weeks. No rupture was observed in the healed skin deficit region.

Case 5: A woman of 56 years old was subjected to ablation of her left breast and clearing of her left axillary lymph node because of cancer of her left breast. Since one week after operation, irradiation with an X-ray of 3,000 R was conducted while simultaneously using carmofur as the carcinostatic. The skin tension was strong because of the major operation so that incomplete skin suture partially occurred with the skin transection. Since the healing tendency was hardly observed, she was put in our charge three and half month after operation. The surroundings about the suture line were brown because of the state of radiodermatitis and there was a skin deficit of $29 \times 11$ mm in the middle. The granulation tissue in the wound was dry and dark red. It was believed to be a typical "bad granulation tissue." The therapy with a ubidecarenone ointment was immediately started. After 4 days, the granulation tissue turned scarlet, and regeneration of a light purple skin was observed in the edge region. Healing progressed rapidly so that the skin deficit was reduced to $22 \times 10$ mm after one week, and to $14 \times 9$ mm after 5 weeks. She left the hospital on this day as she desired it. This is a case where the healing was retarded because of various healing-obstructing conditions such as postoperative irradiation and skin tension in the affected region, and hence was believed to be attained only by skin graft. Even in such a case, the healing of the region of the skin deficit could be realized.

Case 6: A woman of 47 years old was subjected to ablation of both of her breasts after irradiation with an X-ray of 3,000 R before the operation because of cancer of both the breasts. The tension of the chest wall was strong and particularly the healing of the operation wound was retarded in the right precordia, so that the hyperbaric oxygen therapy was conducted for one month. However, amelioration was not remarkable. Thus, she was put in our charge after about two months. There was a suture line straighly crossing the left and right precordias, and incomplete suture regions along the suture line and three large necrosis focuses. The therapy with a ubidecarenone ointment was continued for one week. The skin wound turned scarlet so that the healing tendency was observed. Since it was believed that the presence of a sphacelus prevented the healing mechanism, the sphacelus was removed with a surgical knife. Thereafter, epithelialization progressed satisfactorily and complete healing was achieved 20 days after she was put in our charge.

Case 7: A womn of 68 years old was subjected to ablation of her left breast and clearing of her left axillary lymph node seven years ago because of cancer of her left breast. One year ago, she was subjected to cryosurgery because of local relapse. However, because of further relapse, she was put in our charge. She was subjected to irradiation with an X-ray of 4,000 R while simultaneously using a Mifurol ointment. Since radiodermatitis appeared, she took a cytochrome c preparation to heal the same. She fell down to strike her left precordia with a corner of a table. Since formation of an ulcer was observed, she was put in our charge again. The left precordia was strained and atrophic due to the operation and the radiotherapy and had an ulcer of $12 \times 8$ mm. The therapy with internal use of cytochrome c and subsequently the therapy with a Benzaduck ointment were conducted, and the combined therapy thereof was also conducted. Since any healing tendency was not observed, the therapy with a ubidecarenone ointment was conducted instead. On the same day, a pain mitigated and disappeared. After two days, a reduction of the ulcer became apparent and "good granulation tissue" was observed. Thereafter, healing progressed satisfactorily. After 3 weeks, the ulcer was completely healed. This also was a striking healing effect

Case 8: A female baby of 6 months old was struck in the face with a corner of a cabinet while playing and underwent a contused wound in the left side of the root of her nose. The wound was about 3 mm × 3 mm in size but considerably deep. Healing was retarded because she was a baby and the wound was positioned in the middle of her face so that sufficient cleanliness was not kept in the wound. Thus, a little swelling of keloid remained. When a ubidecarenone ointment was repeatedly applied to the wound, the keloid was absorbed and disappeared.

The following conclusions were drawn from the foregoing Experimental Example and Cases.

(A) The characteristic feature of the remedy of the present invention consists in notable promotion of cuticle formation (epithelialization). It was observed that, while a "bad granulation tissue" was turned into a "good granulation tissue," cuticle formation started and progressed.

(B) The course of healing of a wound comprises (I) a deficit of the skin tissue, a strong inflammation reaction and a pain caused by undergoing a wound; (2) a reaction of subcutaneous connective tissue (glanulation); repair of a deficit of the skin by regeneration of epithelium (cuticle). Actually, however, retardation or abortion of healing occurs since one or more of steps (I), (2), and (3) are obstructed by various causes such as local dirtiness, repeated bruises, a previous treatment, e.g. radiotherapy, and aging. It became apparent that application of the remedy of the present invention improved the healing mechanism and promoted the healing of a wound.

One of the effect is mitigation or disappearance of a pain, which could be clearly recognized from 10 to 20 minutes after the start of the therapy. This is probably because the remedy of the present invention instantaneously ameliorates the local reaction, namely edema and ischemia. It is analogous to a phenomenon that the malagma therapy ameliorates a local circulation behavior to promote supply of oxygen, whereby ATP production is finally promoted. Thus, the above-mentioned effect is believed to belong to the effect of cytochrome c in a broad sense.

(C) The reaction of subcutaneous connective tissue is granulation and advance of substitution of fibrous connection. In the first half of the reaction, a good blood circulation state is necessary. If in an ischemic state, propagation of fibroblast is promoted under the condition of a low oxygen concentration to form a "bad granulation tissue," and, in an extreme case, keloid formation (excessive fibroma formation) occurs. In this case, it became apparent that the remedy of the present invention ameliorated blood circulation to suppress excessive formation of a fibrous connective tissue and form a "good granulation tissue."

In general, an operation wound involves a number of causes of local ischemia such as injury of a local vasculature and skin tension. Particularly, in an operation wound after radiotherapy, local ischemia is severe after irradiation. Thus, it is very difficult to cure the wound. However, the remedy of the present invention can overcome the barrier of local ischemia and can be expected to provide a curing effect.

(D) It is a matter of common knowledge that a cicatricial keloid, once formed, can not be absorbed. However, it was observed that the remedy of the present invention can absorb a cicatricial keloid of a certain severity and cure the same. This may be partially because capillaries are regenerated by the remedy of the present invention while at the same time a fibrinolysin is increasedly formed and secreted.

As described above in (A) to (D), it has been found that the remedy of the present invention, when administered to the skin, promotes the healing of a fresh wound involving a skin deficit, induces healing of a healing mechanism-obstructed wound, and shows an effect of preventing keloid formation and promoting keloid absorption. It has been also found that the remedy of the present invention, which is designed for percutaneous administration, is preferably in the form of a lotion rather than an ointment for operation wounds and that spraying over the operation wound or application of a gauze impregnated with ubidecarenone to the operation wound is preferable. During the therapy in the aforementioned cases, none of fervescence, eqiption or exacerbation of a rash, eruption of allergosis, eruption of other local abnormal reaction and systemic adverse reactions such as an anaphylactic shock were observed. Thus, it is apparent that the remedy of the present invention can be safely and repeatedly used.

**Claims**

Use of ubidecarenone for preparation of a wound remedy.